# EUROPEAN PATENT APPLICATION

(11) **EP 4 509 213 A1**
(43) Date of publication of application: **19.02.2025**
(21) Application number: 23783710.9
(22) Date of filing: 27.06.2023
(51) Int. Cl.: B01L 3/00, C12N 15/10

(54) **APPARATUS AND METHOD FOR ISOLATING NUCLEIC ACID**

(30) Priority: 23.06.2023 KR 20230081163
(71) Applicant: Genesystem Co., Ltd., Yuseong-gu Daejeon 34028 (KR)
(72) Inventor: PARK, Jae Hyung, Daejeon 34519 (KR); KIM, Dong Hyun, Sejong 30100 (KR); BAE, Dong Nyuk, Chungcheongbuk-do 28115 (KR); PARK, Jin Hee, Daejeon 34046 (KR); LEE, Do Bu, Chungcheongbuk-do 28165 (KR); PARK, Jun Hee, Daejeon 34207 (KR); PARK, Chan, Daejeon 35045 (KR); CHOI, Ok Ran, Chungcheongbuk-do 28118 (KR); SEO, Yu Jin, Gyeonggi-do 14295 (KR)
(74) Representative: Loyer & Abello
(86) International application number: PCT/KR2023/008918
(87) International publication number: WO 2024/262682

(57) **Abstract**

The present disclosure relates to a nucleic acid isolation device and method. The nucleic acid isolation device includes: a main body formed to allow a biological sample to pass through from top to bottom; and an absorption layer provided inside the main body to absorb an inhibitor that inhibits a polymerase chain reaction (PCR) and is included in the sample, wherein, when the sample passes through the main body, the inhibitor is absorbed into the absorption layer and nucleic acids are discharged through a lower portion of the main body.

## Description

### TECHNICAL FIELD

The present disclosure relates to a nucleic acid isolation device and method for removing foreign substances from a biological sample and quickly and easily isolating nucleic acids.

### BACKGROUND ART

Polymerase chain reaction (hereinafter referred to as PCR) is a method of amplifying a particular target genetic material to be detected. Also, PCR is a technique for making a large amount of copies of genetic material having the same base sequence by amplifying a small amount of genetic material. PCR is used to amplify human deoxyribonucleic acid (DNA) to diagnose various genetic disorders or is applied to DNA of bacteria, viruses, or fungi to diagnose infectious diseases.

In general, PCR includes the following three steps that are performed repeatedly. The three steps include 1) a denaturing step of heating a sample solution containing a double-stranded DNA to a particular temperature, for example, about 95 °C, to separate the double-stranded DNA into single-stranded DNA, 2) after the denaturing step, an annealing step of providing the sample solution with an oligonucleotide primer having a sequence complementary to a particular nucleotide sequence to be amplified, and cooling the primer and the single-stranded DNA to a particular temperature, for example, 55 °C, such that the primer is combined with a particular nucleotide sequence of the single-stranded DNA to form a partial DNA-primer complex, and 3) after the annealing step, an extension (or amplification) step of maintaining the sample solution at an activation temperature of DNA polymerase, for example, 72 °C, such that a double-stranded DNA is formed based on the primer of the partial DNA-primer complex by using the DNA polymerase. By repeating these three steps several times, a target nucleic acid having a particular nucleotide sequence may be exponentially amplified.

To perform PCR, it is necessary to extract nucleic acids from a biological sample.

An example of a nucleic acid extraction technique in the art is a method of solubilizing a sample including cells with sodium dodecyl sulfate (SDS) or proteinase K, denaturing and removing proteins with phenol, and then purifying a nucleic acid. However, the phenol-based extraction method involves a large number of processes, which takes a lot of time, and may lead to a significant decrease in reliability because the efficiency of nucleic acid extraction greatly depends on the operator's experience and skills. Recently, to resolve such issues, a kit using silica or glass fiber that binds specifically to nucleic acids has been used. The silica or glass fiber has low combining ratios with proteins or cell metabolites, and thus may be used to obtain nucleic acids at a relatively high concentration. This method is advantageous in that it is more convenient than the phenol-based method, but uses a chaotropic reagent or ethanol that strongly inhibits enzyme reactions such as polymerase chain reaction (PCR), and thus requires a complete removal of such substances, that is, the chaotropic reagent or ethanol, which makes the method cumbersome and time-consuming.

In addition, Korean Patent No. 10-0454869 discloses DNA extraction using a cell lysis buffer, which is performed as follows. 1) After culturing animal cells, a suspension containing the cells is centrifuged to recover the cells. 2) 300 *µ*ℓ of a cell lysis buffer are added to the recovered cells. 3) The cells are left at 70 °C for 5 minutes after adding the cell lysis buffer. 4) The dissolved cells are pipetted 2 or 3 times to untangle proteins and the like that are denatured in the dissolution process and allow them to pass through a filter. 5) The dissolved cells are transferred to the filter made of a silica membrane, and centrifuged at 13,000 rpm for 1 minute, then the remaining liquid is discarded, and the cells are centrifuged again. 6) 500 *µ*ℓ of a washing buffer are added to the filter, and then the filter is centrifuged. To increase the efficiency, the remaining liquid is discarded and then the filter is centrifuged again to completely remove the washing buffer. 7) 200 *µ*ℓ of an elution buffer are added to the filter, then the filter is centrifuged, and the eluted liquid is centrifuged again with the filter to obtain a larger amount of genomic DNA. 8) The extracted DNA is electrophoresed in a 12 % agarose gel, stained with ethidium bromide (EtBr), and then observed.

As such, the nucleic acid extraction method in the art includes: 1) adding a cell lysis buffer to cells to lyse the cells; 2) transferring the lysed cells of the operation 1) to a filter and fixing nucleic acids; 3) washing the filter of operation 2); and 4) recovering the nucleic acids from the filter, and thus, this method enables extraction of nucleic acids with high reproducibility by performing a small number of operations in a short time. However, a centrifuge is used for transferring the dissolved cells to the filter, washing the filter, or recovering the nucleic acids from the filter. The use of a centrifuge has the effect of shortening the process time, but has an issue of being less portable and mobile and complicating an on-site nucleic acid extraction.

In addition to the above method, there also are a nucleic acid extraction method using magnetic beads, a nucleic acid extraction method using a syringe and a filter, a nucleic acid extraction method using a direct lysis buffer (DLB), and a nucleic acid extraction method using Trizol. However, the nucleic acid extraction method using magnet beads requires a magnet to fix nucleic acids to a wall of a tube and the use of a pump or valve (an automated device), or a plurality of tips and pipettes (manual) to remove liquids. In addition, the nucleic acid extraction method using a syringe and a filter has an issue in that, when a force greater than or equal to a certain value is applied during a process of transferring nucleic acids by using the syringe, the filter is damaged, making extraction of the nucleic acids difficult. The nucleic acid extraction method using a DLB requires dilution of the DLB to 1/10 because PCR inhibitors may be present in the DLB, resulting in a significant decrease in the sensitivity due to the dilution. In addition, the nucleic acid extraction method using Trizol has an issue of using harmful organic solvents such as phenol or chloroform.

Thus, there is a need for a device capable of quickly and easily isolating nucleic acids from a biological sample without using additional equipment.

### DESCRIPTION OF EMBODIMENTS

### TECHNICAL PROBLEM

The present disclosure provides a nucleic acid isolation device and method for quickly and easily isolating nucleic acids by effectively removing foreign substances from a biological sample without using additional equipment.

### SOLUTION TO PROBLEM

A nucleic acid isolation device for a polymerase chain reaction (PCR) according to an embodiment of the present disclosure includes: a main body formed to allow a biological sample to pass through from top to bottom; and an absorption layer provided inside the main body to absorb an inhibitor that inhibits a PCR and is included in the sample, wherein, when the sample passes through the main body, the inhibitor is absorbed into the absorption layer and nucleic acids are discharged through a lower portion of the main body.

In addition, it is preferable that the absorption layer includes at least one resin layer selected from a cation exchange resin layer that is positively charged, an anion exchange resin layer that is negatively charged, or a chelate resin layer.

In addition, it is preferable that the anion exchange resin layer, the chelate resin layer, and the cation exchange resin layer are sequentially arranged in the absorption layer from top to bottom of the main body.

In addition, it is preferable that the absorption layer includes an anion exchange resin layer, a chelate resin layer, and a cation exchange resin layer, and a volume ratio of the anion exchange resin layer, the chelate resin layer, and the cation exchange resin layer is 1:1:1.

In addition, it is preferable that a flow rate at which the sample passes through the inside of the main body is 15 *µ*ℓ/sec or less.

In addition, it is preferable that the nucleic acid isolation device further includes: an input chamber having a space formed therein for inputting the sample into an upper portion of the main body; and a collection chamber having a space formed therein for collecting the nucleic acids discharged from the lower portion of the main body.

In addition, it is preferable that the main body includes an accommodation part in which the input sample is accommodated, an adsorption part in which the absorption layer is provided, and a discharge part formed below the adsorption part.

In addition, it is preferable that an upper filter and a lower filter both formed in a mesh structure to filter foreign substances contained in the biological sample are provided on an upper portion and a lower portion of the absorption layer, respectively.

A nucleic acid isolation method for a PCR test according to another aspect of the present disclosure includes: preparing a sample by injecting, into a biological sample, a lysis buffer that destroys cell walls to allow nucleic acids to leak out; injecting, into an upper portion of a main body, the biological sample from which the nucleic acids are exposed; causing an inhibitor that inhibits a PCR to be absorbed and separated from the biological sample by an absorption layer provided inside the main body to absorb the inhibitor; and collecting the nucleic acids discharged through a lower portion of the main body after the inhibitor is separated from the biological sample.

Here, it is preferable that the absorption layer includes at least one resin layer selected from a cation exchange resin layer that is positively charged, an anion exchange resin layer that is negatively charged, or a chelate resin layer.

Here, it is preferable that, in the absorption layer, the anion exchange resin layer, the chelate resin layer, and the cation exchange resin layer are arranged sequentially from top to bottom of the main body.

Here, it is preferable that the absorption layer includes an anion exchange resin layer, a chelate resin layer, and a cation exchange resin layer, and a volume ratio of the anion exchange resin layer, the chelate resin layer, and the cation exchange resin layer is 1:1:1.

Here, it is preferable that a flow rate at which the sample passes through the inside of the main body is 15 *µ*ℓ/sec or less.

Here, it is preferable that, in the causing of the inhibitor to be absorbed and separated from the biological sample, a first sub-body in which the anion exchange resin layer is provided, a second sub-body in which the chelate resin layer is provided, and a third sub-body in which the cation exchange resin layer is provided are provided separately from each other such that the sample passes sequentially through the first, second, and third sub-bodies.

Here, it is preferable that the lysis buffer is a lysis buffer.

Here, it is preferable that the absorption layer has polarity, the main body is filled with a preservation buffer that maintains the polarity of the absorption layer, and the nucleic acid isolation method further includes washing the main body by discharging the preservation buffer from the main body before inputting the sample into the main body.

Here, it is preferable that the nucleic acids are used for polymerase chain reaction (PCR).

### ADVANTAGEOUS EFFECTS OF DISCLOSURE

A nucleic acid isolation device and method according to embodiments of the present disclosure may effectively remove foreign substances from a biological sample and quickly and easily isolate nucleic acids.

In addition, according to the present disclosure, the use of additional equipment such as a centrifuge for nucleic acid isolation is eliminated, making it easy to carry and move the device to a site, thus improving user convenience.

In addition, according to the present disclosure, when a biological sample moves inside a main body of the device from top to bottom, foreign substances are adsorbed and removed in the main body and nucleic acids are discharged downward and collected such that the amounts of the input sample and extracted nucleic acids are substantially equal to each other, and thus, it is possible to secure a large amount of nucleic acids from which an inhibitor is removed, in a single process.

### BRIEF DESCRIPTION OF DRAWINGS

FIG. 1 is a diagram schematically illustrating a nucleic acid isolation device according to an embodiment of the present disclosure.
FIG. 2 is a diagram illustrating a configuration of an absorption layer according to another embodiment of the present disclosure.
FIG. 3 is a diagram illustrating a nucleic acid isolation device according to another embodiment of the present disclosure.
FIG. 4 is a flowchart of a nucleic acid isolation method according to an embodiment of the present disclosure.
FIG. 5 is a diagram illustrating a sample input process.

### MODE OF DISCLOSURE

Hereinafter, various embodiments of the present disclosure will be described with reference to the accompanying drawings. Various embodiments of the present disclosure may be variously modified and may have various embodiments, and particular embodiments are illustrated in the drawings and detailed descriptions related to the embodiments are described. However, this is not intended to limit various embodiments of the present disclosure to particular modes of practice, and it is to be appreciated that all changes, equivalents, and/or substitutes that do not depart from the spirit and technical scope of various embodiments of the present disclosure are encompassed in the present disclosure. With regard to the description of the drawings, similar reference numerals are used to refer to similar elements.

As used in various embodiments of the present disclosure, the expressions "include", "may include", and other conjugates refer to the existence of a corresponding disclosed function, operation, or constituent element, and do not limit one or more additional functions, operations, or constituent elements. In addition, as used in various embodiments of the present embodiment, the terms "include", "have", and other conjugates are intended merely to denote a certain feature, numeral, step, operation, element, component, or a combination thereof, and should not be construed to initially exclude the existence of or a possibility of addition of one or more other features, numerals, steps, operations, elements, components, or combinations thereof.

It should be understood that, when it is described that an element is "connected" to another element, the first element may be directly connected to the second element, and a third element may be "connected" between the first and second elements. On the other hand, it should be understood that, when it is described that a first element is "directly connected" or "directly coupled" to a second element, no further element is present between the first element and the second element.

The terms used in various embodiments of the present disclosure are used only to describe a particular embodiment, and are not intended to limit the various embodiments of the present disclosure. Singular forms are intended to include plural forms as well, unless the context clearly indicates otherwise.

Unless otherwise defined, all terms, including technical and scientific terms, used herein have the same meaning as commonly understood by those of skill in the art to which the present disclosure pertains based on an understanding of the present disclosure.

Terms, such as those defined in commonly used dictionaries, are to be interpreted as having a meaning that is consistent with their meaning in the context of the relevant art and various embodiments of the present disclosure, and are not to be interpreted in an idealized or overly formal sense unless expressly so defined herein.

The present disclosure relates to a nucleic acid isolation device and method, and relates to a device for removing inhibitors from a biological sample, and isolating and obtaining nucleic acids in order to perform polymerase chain reaction (PCR) on the biological sample. Of course, the nucleic acid isolation device according to the present disclosure may be used not only when extraction of nucleic acids is required for the purpose of diagnosis, treatment, or prevention of a disease, but also when extraction of nucleic acids from a sample is required in various fields such as new drug development or detection of environmental hormones.

First, the term 'PCR' used herein refers to a reaction in which a particular target nucleic acid molecule is amplified by using a thermostable deoxyribonucleic acid (DNA) polymerase. In PCR, in addition to the DNA polymerase, a reaction mixture containing divalent ions such as primers (e.g., forward primers or reverse primers), deoxynucleotide triphosphate (dNTP) mixtures, or Mg2+, which are oligonucleotides that may hybridize specifically to a target nucleic acid, may be used.

The 'primer' is used to initiate a PCR reaction, and refers to an oligonucleotide or polynucleotide that hybridizes complementary to template DNA. As the primer for the PCR reaction, a pair of a forward primer (or a sense primer) selected from sense strands in the same direction as that of a genetic code of a nucleic acid molecule to be amplified, and a reverse primer (or an antisense primer) selected from antisense strands complementary to the sense strands may be used.

The 'sample' refers to a genetic material to be amplified, such as a nucleic acid, or a biological solution containing such a genetic material. In addition, the 'reagent' is for detecting the target genetic material and may include a fluorescent dye, a primer, or the like. The primer may consist of a pair of primers with a length of 15 bp to 30 bp that may be bound to both ends of a particular region of a target gene. In addition, as the DNA polymerase, an enzyme that does not lose its activity even at a high temperature of 90 °C or higher may be used.

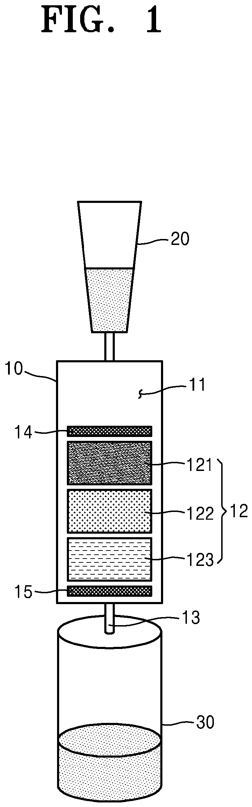

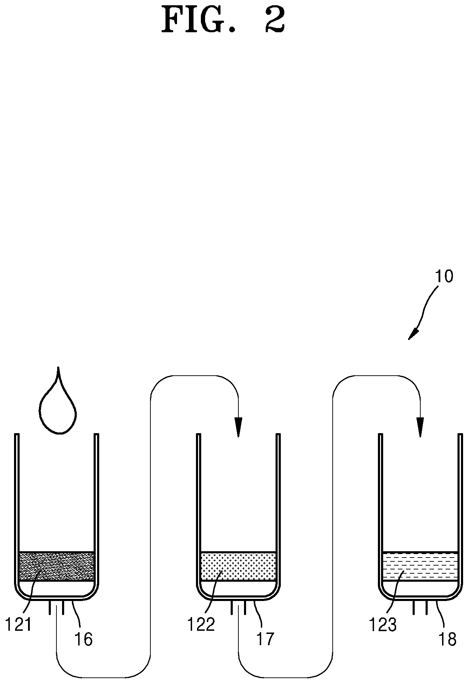

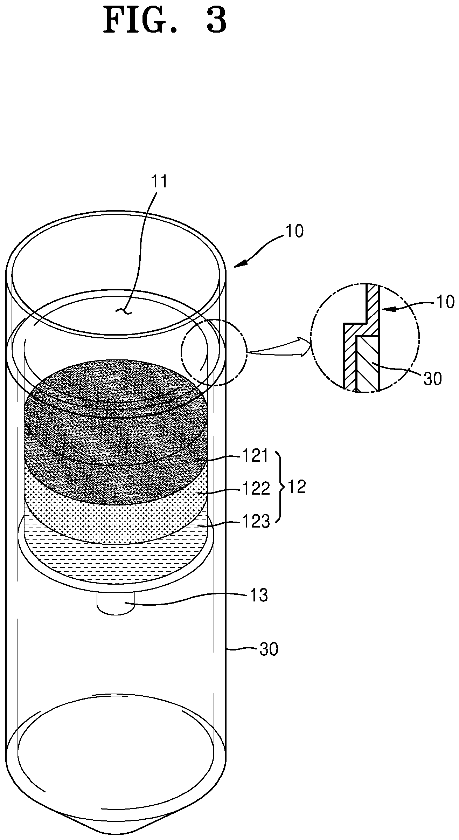

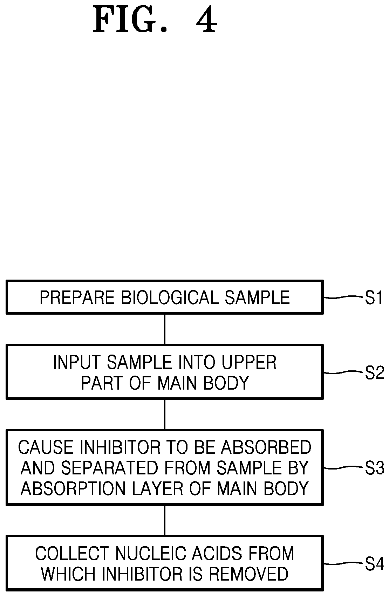

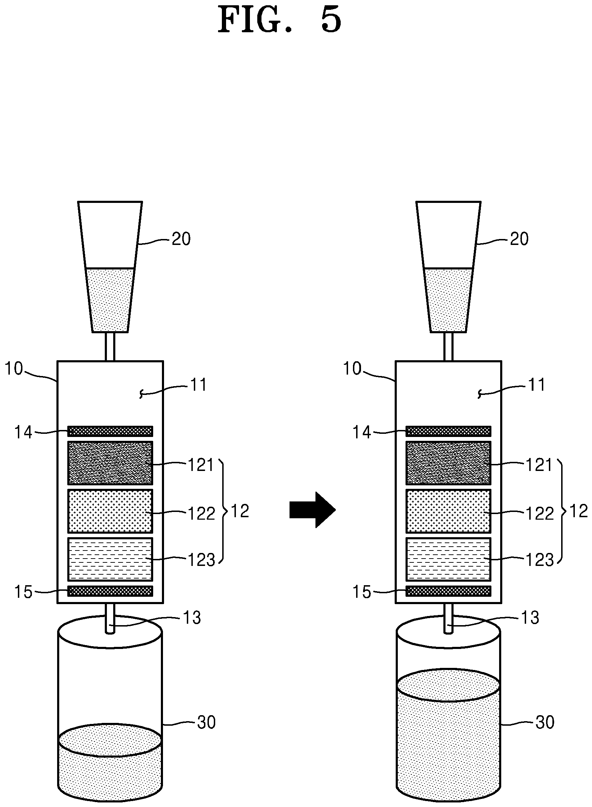

FIG. 1 is a diagram schematically illustrating a nucleic acid isolation device according to an embodiment of the present disclosure, FIG. 2 is a diagram illustrating a configuration of an absorption layer according to another embodiment of the present disclosure, and FIG. 3 is a diagram illustrating a nucleic acid isolation device according to another embodiment of the present disclosure. FIG. 4 is a flowchart of a nucleic acid isolation method according to an embodiment of the present disclosure, and FIG. 5 is a diagram illustrating a sample input process.

Hereinafter, preferred embodiments of the present disclosure will be described with reference to the accompanying drawings.

The nucleic acid isolation device according to an embodiment of the present disclosure includes a main body 10 and an absorption layer 124 provided inside the main body 10, such that, when a biological sample passes through the main body 10, an inhibitor that inhibits a PCR is absorbed into the absorption layer 124, and nucleic acids are discharged through a lower portion of the main body 10 to isolate the nucleic acids from the sample.

The main body 10 is formed such that the biological sample passes therethrough from top to bottom. The main body 10 may be provided in the form of a pipe or a tube that is open in the vertical direction. The main body 10 may be made of materials such as glass, polycarbonate (PC), polymethyl methacrylate (PMMA), polypropylene (PP), polyethylene (PE), metal, or a synthetic resin. The main body 10 may have a cylindrical shape as a whole.

According to the present embodiment, the main body 10 includes an accommodation part 11, an adsorption part 12, and a discharge part 13.

The accommodation part 11 provides a space in which the input biological sample is accommodated. When the biological sample is input, the biological sample slowly moves downward inside the main body 10, and thus, the accommodation part 11 provides a space in which the input sample temporarily stays. There are no particular restrictions on the size and shape of the space as long as the space has a sufficient size to accommodate the volume corresponding to a single input of the sample for isolating a required volume of nucleic acid.

The adsorption part 12 is provided inside the main body 10 and includes the absorption layer 124 that absorbs and removes an inhibitor that is included in the sample and inhibits a PCR. The inhibitor includes salts, proteins, and other intracellular chemicals included in the sample, acts as a factor that interferes with amplification of nucleic acids due to the substances when performing PCR, and is removed by the absorption layer 124 of the adsorption part 12. The inhibitor contained in the sample may be adsorbed and removed by the absorption layer 124, and only nucleic acids may be discharged downward to be collected, and thus, the nucleic acids may be isolated easily and quickly. The present disclosure provides a novel isolation device and method for isolating nucleic acids, without performing an existing process of fixing nucleic acids by using a certain adsorption filter or the like and reisolating the nucleic acids from the adsorption filter to isolate the nucleic acids from a sample.

The absorption layer 124 may include at least one resin layer selected from an anion exchange resin layer 121 that is negatively charged, a cation exchange resin layer 123 that is positively charged, and a chelate resin layer 122. The chelate resin layer 122 is slightly positively charged and has a property of adsorbing (capturing) divalent metal ions. In addition, each resin layer includes a spherical resin, and the inhibitor is adsorbed on the spherical resin. The diameter of the spherical resin may be about 0.18 mm to about 0.3 mm.

The absorption layer 124 has polarity such that a polar inhibitor in the biological sample may be absorbed into the absorption layer 124 and removed. The absorption **layer 124** may be formed by any one or a combination of the anion exchange resin layer 121, the cation exchange resin layer 123, and the chelate resin layer 122.

According to the present embodiment, the adsorption part 12 includes a plurality of layers, and as illustrated in FIG. 1, the absorption layer 124 includes the anion exchange resin layer 121, the chelate resin layer 122, and the cation exchange resin layer 123. According to the present embodiment, the anion exchange resin layer 121, the chelate resin layer 122, and the cation exchange resin layer 123 are arranged sequentially from top to bottom. It is preferable that the volumes of the anion exchange resin layer 121, the chelate resin layer 122, and the cation exchange resin layer 123 are substantially in a ratio of 1:1:1. Of course, the volume ratio of the anion exchange resin layer 121, the chelate resin layer 122, and the cation exchange resin layer 123 is not limited to 1:1:1. The substances included in the biological sample has polarity and thus may be absorbed into each layer while passing through the anion exchange resin layer 121, the chelate resin layer 122, and the cation exchange resin layer 123, and only nucleic acids may flow downward. The anion exchange resin layer 121 may include at least one of trimethylamine (TMA), dimethylethanolamine (DMEA), tertiary amine, and the like, and the chelate resin layer 122 may include at least one of a carboxyl group (-COOH), iminodiacetic acid chelate, and the like. In addition, the cation exchange resin layer 123 may include at least one of a sulfuric acid group (-SO3H), a carboxyl group (-COOH), a sulfonic acid group (-SO3H), and the like.

The discharge part 13 is formed below the adsorption part 12, and the remaining nucleic acids after the inhibitor is removed from the sample is discharged downward. According to the present embodiment, the sample moves inside the main body 10 at a speed of 15 *µ*ℓ/sec or less, and accordingly, the flow rate of the nucleic acids discharged through the discharge part 13 is 15 *µ*ℓ per second or less. The flow rate per unit time of the nucleic acids discharged through the discharge part 13 may be adjusted by appropriately forming the cross-sectional area of the discharge part 13 through which the nucleic acids pass. As the cross-sectional area of the discharge part 13 relatively increases, the discharge amount of the nucleic acids may increase, but the rate at which the sample passes through the inside of the main body 10 relatively increases, and thus, it may be impossible to secure a time period for the inhibitor to be sufficiently adsorbed on the absorption layer 124. Thus, it was confirmed that the inhibitor could be sufficiently removed by the absorption layer 124 when the discharge flow rate of the nucleic acids was adjusted to 15 *µ*ℓ per second or less.

Meanwhile, according to another embodiment of the present disclosure, the main body 10 may include a sub-body including one absorption layer 124. The sub-body may be provided in plurality. For example, as illustrated in FIG. 2, the main body 10 may include first, second, and third sub-bodies 16, 17, and 18 separated from each other. The first sub-body 16 may include the anion exchange resin layer 121. The second sub-body 17 may include the chelate resin layer 122. The third sub-body 18 may include the cation exchange resin layer 123. In addition, according to the present embodiment, as illustrated in FIG. 2, the sample may first pass through the first sub-body 16 including the anion exchange resin layer 121, and then sequentially pass through the second sub-body 17 including the chelate resin layer 122, and the third sub-body 18 including the cation exchange resin layer 123. A pumping unit may be used to transfer the sample to the adjacent sub-body. When the first, second, and third sub-bodies 16, 17, and 18 are arranged spaced apart from each other, the flow rate of the nucleic acids discharged from the third sub-body 18 is adjusted to be 15 µl per second or less such that the inhibitor may be sufficiently adsorbed and removed in each sub-body. When using the sub-bodies spaced apart from each other, the discharge flow rate of the nucleic acids may be adjusted by the pumping unit. When the main body 10 includes the first, second, and third sub-bodies 16, 17, and 18 as described above, only one absorption layer 124 may be formed in the sub-bodies without the need to stack a plurality of absorption layers 124, and thus, manufacturing convenience may be improved.

According to the present embodiment, the nucleic acid isolation device further includes an input chamber 20, a collection chamber 30, an upper filter 14, and a lower filter 15.

The input chamber 20 is arranged on the main body 10 to provide a space into which the sample is input. That is, the sample may be input into the input chamber 20. As illustrated in FIG. 1, the input chamber 20 is provided on the main body 10, and the input chamber 20 and the main body 10 may be connected to each other by a connection pipe. Referring to FIG. 1, the width of the input chamber 20 decreases toward the bottom such that the sample may be easily input from the top thereof and quickly discharged from the bottom thereof. However, the shape of the input chamber 20 is not limited to the above and may be modified in various ways.

The collection chamber 30 provides a space in which the nucleic acids discharged from the lower portion of the main body 10 are collected. As illustrated in FIG. 1, the nucleic acids falling from the discharge part 13 of the main body 10 are collected in the inner space of the collection chamber 30. The collection chamber 30 provides a space for collecting the nucleic acids, and the shape thereof may be modified in various ways. For example, as illustrated in FIG. 3, the collection chamber 30 may be provided in combination with the main body 10. Referring to FIG. 3, the collection chamber 30 may have a cylindrical structure with an open top, and the main body 10 may be provided with a step in the outer circumferential direction to engage with an edge of the collection chamber 30. An upper portion of the step may engage with the upper circumference of the collection chamber 30, and a lower portion of the step may be inserted into the collection chamber 30, such that the collection chamber 30 is coupled to main body 10. In this case, the accommodation part 11 on the main body 10 may perform the function of the input chamber 20.

The upper filter 14 and the lower filter 15 are provided to filter foreign substances contained in the biological sample, such as cell pieces or fragments. The upper filter 14 is provided on the absorption layer 124, and the lower filter 15 is provided above the absorption layer 124. As illustrated in FIG. 1, the upper filter 14 is coupled to an upper portion of the anion exchange resin layer 121, and the lower filter 15 is coupled to a lower portion of the cation exchange resin layer 123. The foreign substances contained in the sample are primarily filtered by the upper filter 14, thus move to the absorption layer 124, and are secondarily filtered by the lower filter 15 before passing through the absorption layer 124 and falling into the collection chamber 30, such that only nucleic acids are collected in the collection chamber 30.

Hereinafter, operations and effects of the nucleic acid isolation device according to the above configuration will be described based on experimental results.

First, it was confirmed that, when the sample did not include an inhibitor and PCR was performed after passing nucleic acids through a polar resin layer, there was little effect on the number of repetitions of a PCR cycle for detecting a particular nucleotide sequence in a case of performing PCR using a DLB prepared by using a lysis buffer in the art, and in a case of performing PCR after passing the nucleic acids through at least one absorption layer 124. The PCR cycle is defined as the following three steps. The three steps are 1) a denaturing step of heating a sample solution containing a double-stranded DNA to a particular temperature, for example, about 95 °C, to separate the double-stranded DNA into single-stranded DNA, 2) an annealing step of, after the denaturing step, providing the sample solution with an oligonucleotide primer having a sequence complementary to a particular nucleotide sequence to be amplified, and cooling the primer and the single-stranded DNA to a particular temperature, for example, 55 °C, such that the primer is combined with a particular nucleotide sequence of the single-stranded DNA to form a partial DNA-primer complex, and 3) an extension (or amplification) step of, after the annealing step, maintaining the sample solution at an activation temperature of DNA polymerase, for example, 72 °C, such that a double-stranded DNA is formed based on the primer of the partial DNA-primer complex by using the DNA polymerase. The PCR exponentially amplifies a target nucleic acid having a particular nucleotide sequence by repeating the above three steps several times to the extent that detection of the particular nucleotide sequence is possible. In Table 1 below, a cycle threshold (CT) value of 29.56 means that the average number of repetitions of the above cycle is 29.56. It was confirmed from these experimental results that the nucleic acids were slightly negatively charged, but when PCR was performed by passing the nucleic acids through each of the anion exchange resin layer 121, the chelate layer, and the cation exchange resin layer 123, and when PCR was performed by passing the nucleic acids through the absorption layers 124 in three stages, the number of repetitions of the PCR cycle that enables detection of a particular gene was slightly different from that in the method in the art, and thus, the nucleic acids was not affected by the resin layers.

**[Table 1]**

| | CT value of clinical sample without inhibitors |
|---|---|
| DLB | 29.56 |
| Cation exchange resin layer 123 | 29.84 |
| Chelate resin layer | 29.77 |
| Anion exchange resin layer 121 | 30.15 |
| Three-stage resin layers of anion, chelate, and cation | 30.63 |

In addition, when the sample includes an inhibitor, as shown in Table 2 below, the sample prepared by using the lysis buffer in the art included an inhibitor, and thus, even when the above three steps for PCR were performed, the particular nucleotide sequence was not amplified due to the inhibitor. On the contrary, as shown in Table 2, it was confirmed that, when the three steps for PCR were repeatedly performed after passing the sample through the anion exchange resin layer 121, the chelate resin layer 122, and the cation exchange resin layer 123, the particular nucleotide sequence was amplified to a detectable level. In particular, it was confirmed that, when the absorption layer 124 was formed by stacking the anion exchange resin layer 121, the chelate resin layer 122, and the cation exchange resin layer 123 from top to bottom as illustrated in FIG. 1, the number of repetitions of the PCR cycle for detecting the particular nucleotide sequence was the lowest. That is, when the absorption layer 124 is formed by stacking the three resin layers, amplification of a particular nucleotide sequence may be rapidly completed.

**[Table 2]**

| | CT value of clinical sample with inhibitor |
|---|---|
| DLB (control) | 0 |
| Cation exchange resin layer 123 | 36.68 |
| Chelate resin layer | 41.34 |
| Anion exchange resin layer 121 | 39.56 |
| Three-stage resin layers of anion, chelate, and cation | 35.13 |

According to another aspect of the present disclosure, a nucleic acid isolation is provided.

As illustrated in FIG. 4, the nucleic acid isolation method according to the present embodiment includes preparing a sample by injecting, into a biological sample, a lysis buffer that destroys cell walls to allow nucleic acids to leak out (S1), injecting, into the upper portion of the main body 10, the biological sample from which the nucleic acids are exposed (S2), causing an inhibitor that inhibits a PCR to be absorbed and separated from the biological sample by the absorption layer 124 provided inside the main body 10 to absorb the inhibitor (S3), and collecting the nucleic acids discharged through the lower portion of the main body 10 after the inhibitor is separated from the biological sample (S4).

The preparing of the sample (S1) is for injecting a certain lysis buffer into the biological sample to destroy cell walls to allow nucleic acids to leak out. The lysis buffer is used to destroy cell walls. That is, in the preparing of the sample (S1), cells are lysed by using the lysis buffer to elute intracellular nucleic acids.

The inputting of the sample (S2) is for inputting the sample from which the nucleic acids are exposed, into the upper portion of the main body 10, and is performed by inputting the sample into the input chamber 20. Of course, when the accommodation part 11 of the main body 10 substitutes for the function of the input chamber 20 as illustrated in FIG. 3, the sample may be input into the accommodation part 11.

The causing of the inhibitor to be absorbed and separated (S3) is for causing the inhibitor to be absorbed and removed by the absorption layer 124 provided inside the main body 10. The main body 10 and the absorption layer 124 provided therein may be configured as illustrated in FIG. 1.

According to the present embodiment, as described above, the absorption layer 124 may include at least one resin layer selected from the cation exchange resin layer 123 that is positively charged, the anion exchange resin layer 121 that is negatively charged, or the chelate resin layer 122. That is, the absorption layer 124 may include one selected from the above resin layers, or a combination of two or three of the resin layers.

According to the present embodiment, the anion exchange resin layer 121, the chelate resin layer 122, and the cation exchange resin layer 123 are arranged sequentially in the absorption layer 124 from top to bottom of the main body 10. That is, the absorption layer 124 is composed of three layers of resin, and the volume ratio of the anion exchange resin layer 121, the chelate resin layer, and the cation exchange resin layer 123 is substantially 1:1:1. In addition, the flow rate at which the sample passes through the inside of the main body 10 is adjusted to be 15 *µ*ℓ/sec or less. As the flow rate of the sample passing through the inside of the main body 10 is adjusted to be 15 *µ*ℓ/sec or less, the flow rate of the nucleic acids discharged through the lower portion of the main body 10 is adjusted to be 15 *µ*ℓ per second or less, and the inhibitor is sufficiently absorbed when the sample passes through the absorption layer 124. When the discharge flow rate of the nucleic acids is greater than 15 *µ*ℓ per second, the movement speed of the sample increases and thus the inhibitor is not sufficiently absorbed.

In addition, according to another embodiment of the present disclosure, the first sub-body 16 including the anion exchange resin layer 121, the second sub-body 17 including the chelate resin layer 122, and the third sub-body 18 including the cation exchange resin layer 123 may be provided separately from each other to sequentially pass the sample through the first, second, and third sub-bodies 16, 17, and 18 in the causing of the inhibitor to be absorbed and separated (S3). The first, second, and third sub-bodies 16, 17, and 18 are arranged spaced apart from each other, and the sample that has passed through the first sub-body 16 may be transferred to the second sub-body 17 to pass therethrough, and then pass through the third sub-body 18. In this case, in the collecting of the nucleic acids (S4), the nucleic acids may be collected at the rear end of the third sub-body 18.

In the collecting of the nucleic acids (S4), when the inhibitor is absorbed and removed in the absorption layer 124, the nucleic acids are discharged through the lower portion of the main body 10, and the discharged nucleic acids are collected. According to an embodiment of the present disclosure, when 500 *µ*ℓ of the biological sample is injected, the inhibitor included in the sample is removed and the flow rate of the sample discharged through the lower portion of the main body 10 is maintained at approximately 500 *µ*ℓ, includes only the nucleic acids without the inhibitor. This significantly improves the flow rate of the sample including only the nucleic acids compared to that in the art.

In addition, according to an embodiment of the present disclosure, the nucleic acid isolation method further includes washing the main body 10 before inputting the biological sample into the main body 10.

The left side of FIG. 5 illustrates washing the main body 10 by primarily discharging a preservation buffer that maintains the polarity of the absorption layer 124. According to an embodiment of the present disclosure, the absorption layer 124 is made of a resin layer having polarity, and the polarity of the absorption layer 124 needs to be maintained until the main body 10 is used. To this end, the inside of the main body 10 is filled with the preservation buffer to maintain the polarity of the absorption layer 124.

When using the nucleic acid isolation device according to the present disclosure, the preservation buffer is discharged. As illustrated on the left side of FIG. 5, a washing buffer (e.g., water) is input into the input chamber 20 to discharge the preservation buffer inside the main body 10. Next, the right side of FIG. 5 illustrates a state in which the sample is input after washing the preservation buffer, and when the sample is input, the washing buffer filled in the main body 10 is first discharged to fill the collection chamber 30 below the main body 10. After all of the washing buffer is pushed out by the input sample and thus discharged, the nucleic acids being discharged are collected below the main body 10.

As such, the nucleic acid isolation device and method according to embodiments of the present disclosure may quickly and easily secure nucleic acids by effectively removing an inhibitor from a biological sample. They provide an effect of significantly shortening a time period required for PCR by quickly securing nucleic acids. In addition, according to the present disclosure, the use of additional equipment such as a centrifuge for nucleic acid isolation is eliminated, making it easy to carry and move the device to a site and improving user convenience.

The present disclosure is described above in detail with reference to preferred embodiments, but the present disclosure is not limited to the above embodiments, and various modifications may be made without departing from the scope of the present disclosure.

## Claims

1. A nucleic acid isolation device comprising:
a main body (10) formed to allow a biological sample to pass through from top to bottom; and
an absorption layer (124) provided inside the main body (10) to absorb an inhibitor that inhibits a polymerase chain reaction (PCR) and is included in the sample,
wherein, when the sample passes through the main body (10), the inhibitor is absorbed into the absorption layer (124) and nucleic acids are discharged through a lower portion of the main body (10).

2. The nucleic acid isolation device of claim 1, wherein the absorption layer (124) comprises at least one resin layer selected from a cation exchange resin layer (123) that is positively charged, an anion exchange resin layer (121) that is negatively charged, or a chelate resin layer (122).

3. The nucleic acid isolation device of claim 1, wherein the anion exchange resin layer (121), the chelate resin layer (122), and the cation exchange resin layer (123) are arranged sequentially in the absorption layer (124) from top to bottom of the main body (10).

4. The nucleic acid isolation device of claim 1, wherein the absorption layer (124) comprises an anion exchange resin layer (121), a chelate resin layer (122), and a cation exchange resin layer (123), and
a volume ratio of the anion exchange resin layer (121), the chelate resin layer (122), and the cation exchange resin layer (123) is 1:1:1.

5. The nucleic acid isolation device of claim 1, wherein a flow rate at which the sample passes through the inside of the main body (10) is 15 *µ*ℓ/sec or less.

6. The nucleic acid isolation device of claim 1, further comprising:
an input chamber (20) having a space formed therein for inputting the sample into an upper portion of the main body (10); and
a collection chamber (30) having a space formed therein for collecting the nucleic acids discharged from the lower portion of the main body (10).

7. The nucleic acid isolation device of claim 1, wherein the main body (10) comprises an accommodation part (11) in which the input sample is accommodated, an adsorption part (12) in which the absorption layer (124) is provided, and a discharge part (13) formed below the adsorption part (12).

8. The nucleic acid isolation device of claim 1, wherein an upper filter (14) and a lower filter (15) both formed in a mesh structure to filter foreign substances contained in the biological sample are provided on an upper portion and a lower portion of the absorption layer (124), respectively.

9. A nucleic acid isolation method comprising:
preparing a sample by injecting, into a biological sample, a lysis buffer that destroys cell walls to allow nucleic acids to leak out;
injecting, into an upper portion of a main body (10), the biological sample from which the nucleic acids are exposed;
causing an inhibitor that inhibits a polymerase chain reaction (PCR) to be absorbed and separated from the biological sample by an absorption layer (124) provided inside the main body (10) to absorb the inhibitor; and
collecting the nucleic acids discharged through a lower portion of the main body (10) after the inhibitor is separated from the biological sample.

10. The nucleic acid isolation method of claim 9, wherein the absorption layer (124) comprises at least one resin layer selected from a cation exchange resin layer (123) that is positively charged, an anion exchange resin layer (121)that is negatively charged, or a chelate resin layer (122).

11. The nucleic acid isolation method of claim 9, wherein the anion exchange resin layer (121), the chelate resin layer (122), and the cation exchange resin layer (123) are arranged sequentially in the absorption layer (124) from top to bottom of the main body (10).

12. The nucleic acid isolation method of claim 9, wherein the absorption layer (124) comprises an anion exchange resin layer (121), a chelate resin layer (122), and a cation exchange resin layer (123), and
a volume ratio of the anion exchange resin layer (121), the chelate resin layer (122), and the cation exchange resin layer (123) is 1:1:1.

13. The nucleic acid isolation method of claim 9, wherein a flow rate at which the sample passes through the inside of the main body (10) is 15 *µ*ℓ/sec or less.

14. The nucleic acid isolation method of claim 9, wherein, in the causing of the inhibitor to be absorbed and separated from the biological sample, a first sub-body (16) in which the anion exchange resin layer (121) is provided, a second sub-body (17) in which the chelate resin layer (122) is provided, and a third sub-body (18) in which the cation exchange resin layer (123) is provided are provided separately from each other such that the sample passes sequentially through the first, second, and third sub-bodies (16, 17, and 18).

15. The nucleic acid isolation method of claim 9, wherein the lysis buffer is a lysis buffer.

16. The nucleic acid isolation method of claim 9, wherein the absorption layer (124) has polarity, the main body (10) is filled with a preservation buffer that maintains the polarity of the absorption layer (124), and
the nucleic acid isolation method further comprises washing the main body (10) by discharging the preservation buffer from the main body (10) before inputting the sample into the main body (10).

17. The nucleic acid isolation method of claim 9, wherein the nucleic acids are used for a PCR.
